# EUROPÄISCHE PATENTANMELDUNG

(11) **EP 2 119 790 A1**
(43) Veröffentlichungstag der Anmeldung: **18.11.2009**
(21) Anmeldenummer: 08009054.1
(22) Anmeldetag: 16.05.2008
(51) Int. Cl.: C12P 7/24

(54) **1,2-Addition von Carbonylverbindungen unter Verwendung des Enzyms YerE**

(71) Anmelder: Albert-Ludwigs-Universität Freiburg, 79085 Freiburg (DE)
(72) Erfinder: Müller, Michael, 79194 Gundelfingen (DE); Lehwald, Patrizia, 79211 Denzlingen (DE); Richter, Michael, 79110 Freiburg (DE)
(74) Vertreter: Keller, Günter

(57) **Zusammenfassung**

Offenbart wird ein Verfahren zur Ausbildung einer kovalenten Bindung zwischen zwei Gruppen mit Carbonylresten gemäß der vorliegenden Reaktionsgleichung: 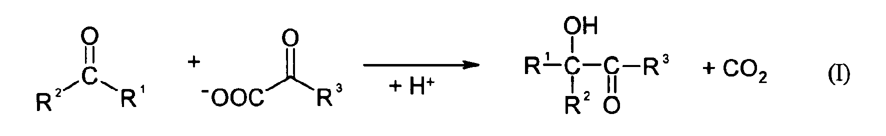 worin
R¹ Wasserstoff, eine Carboxylgruppe oder C₁-C₆ Alkyl ist und
R² ein gegebenenfalls mit Halogen und / oder C₁-C₆ - Alkyl mono-oder disubstituierter aromatischer oder heteroaromatischer Rest oder ein Substituent mit der Formel -AB-C-D ist, wobei A und C jeweils C₁-C₆ Alkyl, Carbonyl oder eine kovalente Bindung ist, B ein Heteroatom, wie Sauerstoff und Schwefel und D ein aliphatischer Rest mit 1 bis 6 Kohlenstoffatomen, ein gegebenenfalls substituierter aromatischer oder heteroaromatischer Rest ist,
oder R¹ und R² zusammen einen cyclischen gegebenenfalls mit einem C₁-C₆ Alkylrest und/oder mit einem Hydroxyrest mono- oder disubstituierten Alkylrest bildet, der gegebenenfalls ein Heteroatom, wie Sauerstoff oder Schwefel enthalten kann, und
R³ Wasserstoff oder C₁-C₆ Alkyl ist,
worin die Reaktion durch das Enzym YerE katalysiert wird.

SEQUENCE LISTING

<110> Albert-Ludwig-Universität

<120> Verfahren zur stereospezifischen Synthese von Verbindungen mit
einem Enzym aus Yersinia

<130> ZEE20071217

<160> 2

<170> PatentIn version 3.5

<210> 1
<211> 1698
<212> DNA
<213> Yersinia pseudotuberculosis

<400> 1
atgaaagcat ctgatgcaat agcgcatgtg ttaagtgcta ataatgtaga ttttggtttt 60

gaattgatcg gtggaatgat tgctcatctt gtggatagca tcaattcatt agggaaaaca 120

aaattagttt ctgtacacca tgagcaggct gcagcatttg cagccggtgg aatagctaga 180

gctacgggta atgaaaaaat aggtttagct ttaggtacta gtggacctgg tgctacaaat 240

ttgataactg gcattgcaga ttgttggtta gatagttctc cttgtatttt tattacaggc 300

caggtcaata ctcatgaact taaggggggg cgtggtatcc ggcagcaagg ttttcaagaa 360

ttagatatag ttagtattgt taaaagtata acaaagtatg catatcagat aaaaagcaaa 420

gatgaaataa ttccatgttt gcaaaatgca atcgatatcg caagaagtgg tagacctggg 480

cctgtgctta ttgatatccc aatggatctg caaagagctg aactagatga tagtataatc 540

aattatctta acaatgaaat agattctaat ctgatatcta aaaaagattc atcatctata 600

tttgatgaga tttttagctt actagagcag tctaaacgac ctgtattttt aattggtggt 660

ggtgcaatac acgaagaaaa attcaatctt tggcaaaaca aaatttctga aaataatata 720

ccccatgtcg ccagtttaaa aggtgcggag aaaactttat caataccagg ctattttggt 780

atggtcggta gctatggtac ccgcactgct aattatgcag tacaaaatgc tgacttggtt 840

attgttctag gtagccgttt agacgtaagg cagaccggtg ctgacacgca agattttgca 900

agaaacgcga aaattgtcca aattgatatt gatattgaac aattagataa tagagtgaaa 960

tgtcaactta atattaatgc atcatgtgag gatttctttt cttattattt acgttcgagg 1020

caatgtaatt cgtataactc ttggttccta caattagaaa acaattggac taaaaatttt 1080

gtagatgaat atcaagattt aaaagtaagt ccatttagcc ttttctccat tctaaataat 1140

gcttttaatg ataaaaatgt ccattacgta actgatgtcg gcaacaatca gatgtgggca 1200

gcccattcat tacgtttagg tgtagggcaa gctattcata attctggagg cttgggtgcg 1260

atgggatttg gcatcccaac agctattggt gtgtcatatg cgacaaacta tcctgttgta 1320

gtaattactg gggatggtgg agctcaattg aatatccaag agctagatat tatatctcgc 1380

gaaaatcgtc ctatactaac aatagtttta aacaataatt ctttaggtat ggtacgtgct 1440

tttcaggaaa tgtattttga cggtagaaat gagtcaacat actggagtgg atatagctca 1500

tcattctcta aaattgggaa agcatataat gttgagtcgt acactgtatt aacagaatgt 1560

gacttttag aaaaagtcaa tgtgtttatt gataatccaa agccaatgct tattgaagtt 1620

ttgatggaag atgcaagaga atgtagacca agattagctt ttggtaaccc aattgataaa 1680

caatttccct ataaatag 1698

<210> 2
<211> 565
<212> PRT
<213> Yersinia pseudotuberculosis

<400> 2

Met Lys Ala Ser Asp Ala Ile Ala His Val Leu Ser Ala Asn Asn Val
1               5                   10                  15

Asp Phe Gly Phe Glu Leu Ile Gly Gly Met Ile Ala His Leu Val Asp
            20                  25                  30

Ser Ile Asn Ser Leu Gly Lys Thr Lys Leu Val Ser Val His His Glu
        35                  40                  45

Gln Ala Ala Ala Phe Ala Ala Gly Gly Ile Ala Arg Ala Thr Gly Asn
    50                  55                  60

Glu Lys Ile Gly Leu Ala Leu Gly Thr Ser Gly Pro Gly Ala Thr Asn
65                  70                  75                  80

Leu Ile Thr Gly Ile Ala Asp Cys Trp Leu Asp Ser Ser Pro Cys Ile
                85                  90                  95

Phe Ile Thr Gly Gln Val Asn Thr His Glu Leu Lys Gly Gly Arg Gly
            100                 105                 110

Ile Arg Gln Gln Gly Phe Gln Glu Leu Asp Ile Val Ser Ile Val Lys
        115                 120                 125

Ser Ile Thr Lys Tyr Ala Tyr Gln Ile Lys Ser Lys Asp Glu Ile Ile
    130                 135                 140

Pro Cys Leu Gln Asn Ala Ile Asp Ile Ala Arg Ser Gly Arg Pro Gly
145                 150                 155                 160

Pro Val Leu Ile Asp Ile Pro Met Asp Leu Gln Arg Ala Glu Leu Asp
                165                 170                 175

Asp Ser Ile Ile Asn Tyr Leu Asn Asn Glu Ile Asp Ser Asn Leu Ile
            180                 185                 190

Ser Lys Lys Asp Ser Ser Ser Ile Phe Asp Glu Ile Phe Ser Leu Leu
        195                 200                 205

Glu Gln Ser Lys Arg Pro Val Phe Leu Ile Gly Gly Gly Ala Ile His
    210                 215                 220

Glu Glu Lys Phe Asn Leu Trp Gln Asn Lys Ile Ser Glu Asn Asn Ile
225                 230                 235                 240

Pro His Val Ala Ser Leu Lys Gly Ala Glu Lys Thr Leu Ser Ile Pro
                245                 250                 255

Gly Tyr Phe Gly Met Val Gly Ser Tyr Gly Thr Arg Thr Ala Asn Tyr
            260                 265                 270

Ala Val Gln Asn Ala Asp Leu Val Ile Val Leu Gly Ser Arg Leu Asp
        275                 280                 285

Val Arg Gln Thr Gly Ala Asp Thr Gln Asp Phe Ala Arg Asn Ala Lys
    290                 295                 300

Ile Val Gln Ile Asp Ile Asp Ile Glu Gln Leu Asp Asn Arg Val Lys
305                 310                 315                 320

Cys Gln Leu Asn Ile Asn Ala Ser Cys Glu Asp Phe Phe Ser Tyr Tyr
                325                 330                 335

Leu Arg Ser Arg Gln Cys Asn Ser Tyr Asn Ser Trp Phe Leu Gln Leu
            340                 345                 350

Glu Asn Asn Trp Thr Lys Asn Phe Val Asp Glu Tyr Gln Asp Leu Lys
        355                 360                 365

Val Ser Pro Phe Ser Leu Phe Ser Ile Leu Asn Asn Ala Phe Asn Asp
    370                 375                 380

Lys Asn Val His Tyr Val Thr Asp Val Gly Asn Asn Gln Met Trp Ala
385                 390                 395                 400

Ala His Ser Leu Arg Leu Gly Val Gly Gln Ala Ile His Asn Ser Gly
                405                 410                 415

Gly Leu Gly Ala Met Gly Phe Gly Ile Pro Thr Ala Ile Gly Val Ser
            420                 425                 430

Tyr Ala Thr Asn Tyr Pro Val Val Val Ile Thr Gly Asp Gly Gly Ala
        435                 440                 445

Gln Leu Asn Ile Gln Glu Leu Asp Ile Ile Ser Arg Glu Asn Arg Pro
    450                 455                 460

Ile Leu Thr Ile Val Leu Asn Asn Asn Ser Leu Gly Met Val Arg Ala
465                 470                 475                 480

Phe Gln Glu Met Tyr Phe Asp Gly Arg Asn Glu Ser Thr Tyr Trp Ser
                485                 490                 495

Gly Tyr Ser Ser Ser Phe Ser Lys Ile Gly Lys Ala Tyr Asn Val Glu
            500                 505                 510

Ser Tyr Thr Val Leu Thr Glu Cys Asp Phe Leu Glu Lys Val Asn Val
        515                 520                 525

Phe Ile Asp Asn Pro Lys Pro Met Leu Ile Glu Val Leu Met Glu Asp
    530                 535                 540

Ala Arg Glu Cys Arg Pro Arg Leu Ala Phe Gly Asn Pro Ile Asp Lys
545                 550                 555                 560

Gln Phe Pro Tyr Lys
                565

## Beschreibung

Für die Synthese von enantiomerenreinen Natur- und Wirkstoffen ist es häufig entscheidend, dass bei den einzelnen Syntheseschritten ein hoher Enantiomeren-Überschuß erzielt wird. Das vorliegende Verfahren betrifft die Reaktion von zwei chemischen Gruppen mit Carbonylresten, nämlich Aldehyd- und Ketongruppen in einer Benzoin-Kondensation unter Ausbildung einer Kohlenstoff-Kohlenstoff-Bindung, wobei die Reaktion durch ein bakterielles Enzym katalysiert wird.

Zu den am längsten bekannten Reaktionen der organischen Chemie gehört die Benzoin-Kondensation, bei der Aldehyde in Acyloine überführt werden. Wird einer der beiden Aldehyde durch ein Keton ausgetauscht, könnten sich auf diese Weise tertiäre Alkohole aufbauen lassen. Da es sich bei chiralen tertiären Alkoholen um Verbindungen handelt, die von großem Interesse für die Synthese von enantiomerenreinen Natur- und Wirkstoffen sind, wurden verschiedene chirale Katalysatoren entwickelt, um die Aldehyd-Keton Benzoin Reaktion asymmetrisch durchführen zu können. Bislang ist jedoch das Problem der intermolekularen nicht-enzymatischen asymmetrischen Acyloinkondensation mit Ketonen als Akzeptoren ungelöst. Ursache hierfür stellt die geringere Carbonylaktivität von Ketonen im Vergleich zu Aldehyden dar, welche bevorzugt zu einer Aldehyd-Aldehyd Benzoin Reaktion und somit zu einer Acyloinbildung führt.

Zwar ist die C-C-Bindungsknüpfung von Aldehyden in 1,2-Position (vgl. Benzoin Kondensation) mit Thiamindiphosphat (ThDP)-abhängigen Enzymen mit guter Ausbeute und gutem Enantiomerenüberschuss möglich, jedoch konnte für klassische ThDP-abhängige Enzyme, wie beispielsweise die Benzaldehydlyase (BAL) aus *Pseudomonas fluorescens* sowie die Pyruvatdecarboxylase (PDC) aus *Saccharomyces cerevisiae*, keine Aldehyd-Keton Benzoin Reaktion gezeigt werden.

Es ist eine Aufgabe der vorliegenden Erfindung, ein neues Verfahren zur Ausbildung einer kovalenten Bindung zwischen zwei Gruppen mit Carbonylresten bereit zu stellen mit einer definierten Stereochemie. Die beiden Gruppen mit Carbonylresten können bevorzugt an verschiedenen Molekülen lokalisiert sein, dann handelt es sich um eine intermolekulare Reaktion. Die beiden Gruppen können aber auch an ein und demselben Molekül vorhanden sein, dann handelt es sich um eine intramolekulare Reaktion.

Mit dem erfindungsgemäßen Verfahren ist es möglich, mit dem Thiamindiphosphat-abhängigen Enzym YerE als Katalysator über eine intermolekulare Aldehyd-Keton Benzoin Reaktion 1,2-Additionsprodukte mit einem Enantiomerenüberschuss von > 10:1 zu erhalten. Hierbei können sowohl cyclische und offenkettige aliphatische Ketone als auch Ketone mit aromatischen und heteroaromatischen Substituenten als Akzeptorsubstrate eingesetzt werden. Das Protein YerE weist somit ein breites Substratspektrum auf.

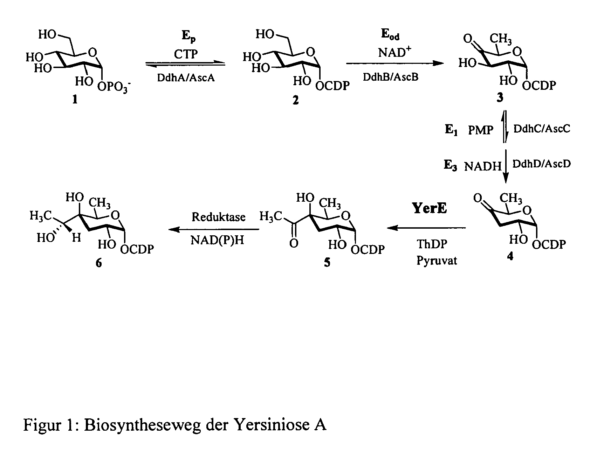

Von Liu et al. (J.Am.Chem.Soc. 1998, S. 11796-11797) wurde 1998 der Biosyntheseweg von Yersiniose A, einer 3,6-Didesoxyhexose publiziert, bei der es sich um einen Bestandteil der Lipopolysaccharide von *Yersinia pseudotuberculosis* O:VI handelt. Das durch *yerE* codierende ThDP-abhängige Enzym katalysiert hierbei sowohl die Decarboxylierung von Pyruvat als auch den Acetyltransfer auf eine aktivierte 3,6-Didesoxy-4-keto-D-glucose. Das Enzym YerE wurde von Liu *et al*. kloniert und mittels Immobilisierter Metallionen Affinitäts-Chromatographie gereinigt. Mittels Gelfiltration wurde für das Homodimer ein molares Gewicht von 117 kDa und für jede Untereinheit eine Größe von 63 kDa bestimmt. Die Funktion des Enzyms YerE bei der Biosynthese der Yersiniose A ist in Figur 1 dargestellt. Zur Bestimmung der Enzymaktivität wurde das Substrat, die aktivierte 3,6-Didesoxy-4-keto-D-glucose, ausgehend von CDP-D-Glucose durch Verwendung der gereinigten Enzyme E_{od}, E₁ und E₃ gewonnen und dessen Umsetzung durch YerE zur Verbindung **5** mittels NMR-Spektroskopie nachgewiesen.

Gegenstand der vorliegenden Erfindung ist ein Verfahren zur Ausbildung einer kovalenten Bindung zwischen zwei Gruppen mit Carbonylresten gemäß der vorliegenden Reaktionsgleichung: 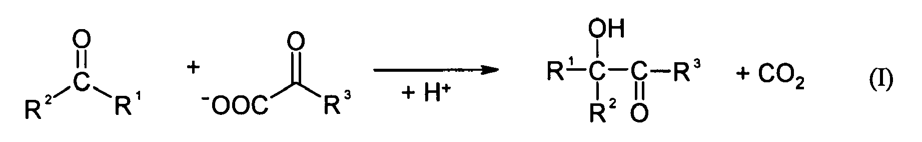 worin
R¹ Wasserstoff, eine Carboxylgruppe oder C₁-C₆ Alkyl ist und
R² ein gegebenenfalls mit Halogen und / oder C₁-C₆-Alkyl mono- oder disubstituierter aromatischer oder heteroaromatischer Rest oder ein Substituent mit der Formel -A-B-C-D ist, wobei A und C jeweils C₁-C₆ Alkyl, Carbonyl oder eine kovalente Bindung ist, B ein Heteroatom, wie Sauerstoff und Schwefel und D ein aliphatischer oder gegebenenfalls substituierter aromatischer oder heteroaromatischer Rest ist,
oder R¹ und R² zusammen einen cyclischen, gegebenenfalls mit einem C₁-C₆ Alkylrest und/oder mit einem Heteroatom mono- oder disubstituierten Alkylrest bildet, der gegebenenfalls ein Heteroatom wie Sauerstoff oder Schwefel enthalten kann, und
R³ Wasserstoff oder C₁-C₆ Alkyl ist, wobei die Reaktion durch das Enzym YerE katalysiert wird.

Das erfindungsgemäß eingesetzte Enzym YerE wurde erstmals bei *Yersinia pseudotuberculosis* beschrieben. Erfindungsgemäß wird bevorzugt ein rekombinant hergestelltes Enzym eingesetzt. Das für YerE kodierende Gen wurde aus *Yersinia pseudotuberculosis* kloniert und wird in geeigneten Wirtsorganismen exprimiert. In bevorzugter Ausführungsform wird das für YerE kodierende Gen in einen geeigneten Expressionsvektor eingebaut und in einem geeigneten Wirtsorganismus exprimiert.

Im Rahmen der vorliegenden Erfindung wurde das für YerE kodierende Gen kloniert und sequenziert. Die für das Protein kodierende Nucleinsäure ist im Sequenzprotokoll als SEQ ID NO:1 offenbart. Im Rahmen der vorliegenden Erfindung können allerdings auch solche YerE-ähnlichen Enzyme eingesetzt werden, die durch ein Gen kodiert werden, das eine ähnliche Sequenz aufweist, wobei allerdings die enzymatische Aktivität zumindest beibehalten bleiben muß.

Dem Fachmann ist bekannt, dass ähnliche Gene auch aus anderen Mikroorganismen isoliert werden. Die vorliegende Erfindung betrifft daher auch die Verwendung von YerE-ähnlichen Enzymen, die auf Nucleotid-Ebene eine Homologie von wenigstens 60 %, bevorzugt wenigstens 80 % und besonders bevorzugt wenigstens 90 %, berechnet über die Gesamtlänge von SEQ ID NO:1, aufweisen.

Dem Fachmann ist auch bekannt, dass die enzymatische Aktivität des Enzyms durch geeignete Mutationen verbessert werden kann. Anhand eines dreidimensionalen Modells der Tertiärstruktur, die mit einem geeigneten Computermodell erstellt werden kann, können diejenigen Aminosäuren, die für die katalytische Aktivität verantwortlich sind, identifiziert werden. Durch schrittweisen Austausch einzelner Aminosäuren kann die enzymatische Aktivität erhöht werden.

SEQ ID NO:2 gibt die Aminosäuresequenz des YerE-Enzyms wieder. Im Rahmen der vorliegenden Erfindung können auch solche Enzyme eingesetzt werden, die eine hohe Ähnlichkeit zu der SEQ ID NO:2 haben. Im Rahmen der vorliegenden Erfindung werden daher für die erfindungsgemäße Umsetzung bevorzugt solche Enzyme eingesetzt, die eine Homologie von wenigstens 70 %, bevorzugt wenigstens 80 % und besonders bevorzugt wenigstens 90 % zu der Aminosäuresequenz mit der Identifikationsnummer 2 aufweisen. Die Homologiewerte beziehen sich dabei auf die Gesamtlänge des in SEQ ID NO:2 wiedergegebenen Proteins.

Für die Durchführung der Reaktion kann das Enzym YerE entweder hochgereinigt oder teilgereinigt werden. Verfahren zum Reinigen eines Enzyms sind dem Durchschnittsfachmann wohlbekannt. Beispielsweise können an die Sequenz kodierend für das Enzym YerE geeignete Tags angefügt werden, die dann mit geeigneten Reinigungsmaterialien wechselwirken und eine effiziente und in den meisten Fällen ausreichende Reinigung in einem Reinigungsschritt ermöglichen. Für die meisten Verwendungszwecke ist es ausreichend, eine rohe Enzympräparation einzusetzen, die bevorzugt etwa 5 bis 80 Gew.-% und besonders bevorzugt 20 bis 80 Gew.-% YerE, bezogen auf den Gesamtproteingehalt, aufweist. Für spezielle Ausführungsformen kann es aber auch empfehlenswert sein, das Enzym höher aufzureinigen, so dass die Proteinpräparation 60 bis 95 % YerE, bezogen auf das gesamte eingesetzte Protein, beinhaltet. Dies kommt insbesondere dann in Betracht, wenn das Enzym in kontinuierlichen Verfahren eingesetzt wird und dabei auf einer festen Phase, beispielsweise Chromatographiematerial, Trägerkügelchen oder ähnlichem immobilisiert wird.

Erfindungsgemäß ist es möglich, durch den Einsatz des Enzyms YerE eine kovalente Bindung zwischen zwei chemischen Gruppen mit Carbonylresten auszubilden. Die zwei Reaktanden der obigen Reaktionsgleichung (I) können als Akzeptorsubstrat und Donorsubstrat angesehen werden.

Bei dem Akzeptor handelt es sich in einer Ausführungsform um eine Verbindung mit der Formel 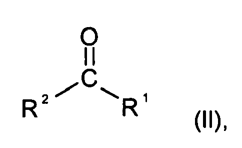 bei der R¹ die Bedeutung Wasserstoff, eine Carboxylgruppe oder C₁-C₆ Alkyl hat und R² ein gegebenenfalls mit Heteroatom und/oder C₁-C₆ Alkyl mono- oder disubstituierter aromatischer Rest ist.

Bei dem Akzeptor der Formel (II) kann es sich auch um Pyruvat handeln. In diesem Fall nimmt der Substituent R¹ die Bedeutung einer Carboxylgruppe und der Substituent R² die Bedeutung einer Methylgruppe ein.

In einer bevorzugten Ausführungsform werden Aldehyde als Akzeptoren eingesetzt. In diesem Fall nimmt der Substituent R¹ die Bedeutung Wasserstoff ein und der Rest R² kann verschiedene Bedeutungen aufweisen. In bevorzugter Ausführungsform kann R² die Bedeutung eines mono- oder disubstituierten aromatischen Restes einnehmen. Hierbei kann es sich um aromatische 5-Ringe oder 6-Ringe sowie kondensierte 5-Ringe und 6-Ringe handeln, die auch Heteroatome wie Sauerstoff, Stickstoff und Schwefel enthalten können. Bevorzugte Beispiele der aromatischen Ringe sind Furan, Benzofuran, Isobenzofuran, Pyrrol, Indol, Isoindol, Thiophen, Benzothiophen, Imidazol, Benzimidazol, Purin, Pyrazol, Indazol, Oxazol, Benzoxazol, Isoxazol, Benzisoxazol, Thioazol, Benzothiazol, Benzol, Naphthalin, Anthracen, Pyridin, Isochinolin, Pyrazin, Chinoxalin, Acridin, Pyrimidin, Chinazolin, Pyridazin oder Cinnolin. Besonders bevorzugt ist Phenyl. Diese aromatischen Reste können mit 1 bis 2 Resten substituiert sein, wobei es sich bei den Substituenten bevorzugterweise um Halogene (F, Cl, Br, I), Hydroxygruppen, C₁-C₆ Alkyl oder C₁-C₆ Alkoxy handelt.

In einer weiteren bevorzugten Ausführungsform handelt es sich bei dem Akzeptor um offenkettige aliphatische Ketone, wobei der Rest R² die Bedeutung C₁-C₆ Alkyl, Carbonyl oder -CH₂XCH₃ oder -CHXCH₂CH₃ aufweist, wobei X die Bedeutung Heteroatom, bevorzugt Schwefel oder Sauerstoff hat.

In einer anderen bevorzugten Ausführungsform handelt es sich bei dem Akzeptor um Ketone mit aromatischen oder heteroaromatischen Substituenten, wobei in der Verbindung mit der Formel 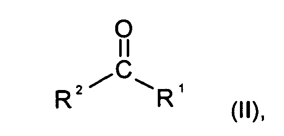 R¹ die Bedeutung C₁-C₆ Alkyl hat und R² ein Substituent mit der Formel -A-B-C-D ist, wobei A und C jeweils die Bedeutung -(CH₂)-ₙ mit n = 1 bis 6 hat oder eine kovalente Bindung ist und D ein mono- oder disubstituierter aromatischer oder heteroaromatischer Rest, wie oben definiert, ist.

In einer bevorzugten Ausführungsform ist R² ausgewählt aus: 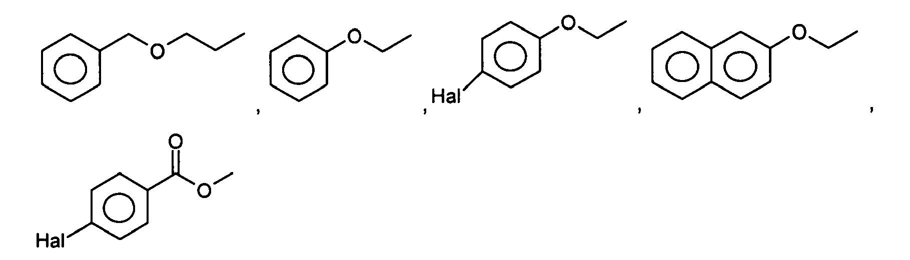 wobei Hal ausgewählt ist aus F, Cl, Br, oder I.

In einer weiteren bevorzugten Ausführungsform können die Substituenten R¹ und R² zusammen einen cyclischen Alkylrest bilden, der gegebenenfalls auch ein Heteroatom wie Schwefel oder Sauerstoff aufweisen kann. Dieser cyclische Alkylrest weist bevorzugt 4-10 Kohlenstoffatome auf. Darüber hinaus kann dieser cyclische Alkylrest mit einem oder mehreren C₁-C₆-Alkylresten oder Heteroatomen substituiert sein, mit der Maßgabe, dass es sich hierbei nicht um 2-Methylcyclohexanon oder 3,3-Dimethylcyclohexanon handelt.

Da das natürliche Substrat des Enzyms YerE Pyruvat ist, handelt es sich bei Pyruvat um ein bevorzugtes Donorsubstrat. Alternativ hierzu kann jedoch das Donorsubstrat als zweiter Reaktand der Reaktionsgleichung (I) eine Verbindung sein mit der Formel: 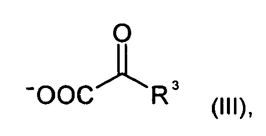 bei der R³ die Bedeutung Wasserstoff oder C₁-C₆ Alkyl hat.

Mit dem Thiamindiphosphat-abhängigen Enzym YerE konnte erstmalig eine katalytische asymmetrische C-C-Bindungsknüpfung eines aktivierten (umgepolten) Aldehyds an ein prochirales Keton gezeigt werden. Dies ist gleichzeitig das erste Beispiel für eine intermolekulare Variante dieser Reaktion.

Für die nicht-enzymatische intermolekulare gemischte Benzoin-Kondensation eines Aldehyds mit einem Keton ist aufgrund der erhöhte Carbonylaktivität des Aldehyds bislang kein Beispiel bekannt. Ausgehend von der in der Literatur publizierten physiologischen Reaktion des Enzyms konnten wir zeigen, dass eine asymmetrische Induktion durch ein chirales Substrat die Induktion durch das Protein übersteigen kann.

Erst durch die Verwendung geeigneter prochiraler Ketone in Kombination mit einer 2-Ketocarbonsäure als Ausgangssubstanz für die Bildung des aktivierten Aldehyds konnten wir die oben genannte Reaktion durch YerE erfolgreich katalysieren. Die hierdurch zugänglichen Enantiomeren angereicherten tertiären Alkohole stellen eine wichtige Gruppe von ansonsten schwer zugänglichen Bausteinen in der Pharmazeutischen und Chemischen Industrie dar.

### Beispiel 1: Bereitstellung des Enzyms YerE

### a) klonierung

Das Ausgangsmaterial für die Arbeiten war die genomische DNA aus *Yersinia pseudotuberculosis O:VI*, erhalten vom Institut für Hygiene und Umwelt Hamburg (Abteilung für Mikrobiologie und Verbraucherschutz). Alternativ hierzu können geeignete Stämme von Stammsammlungen wie der ATCC oder der DSM erhalten werden.

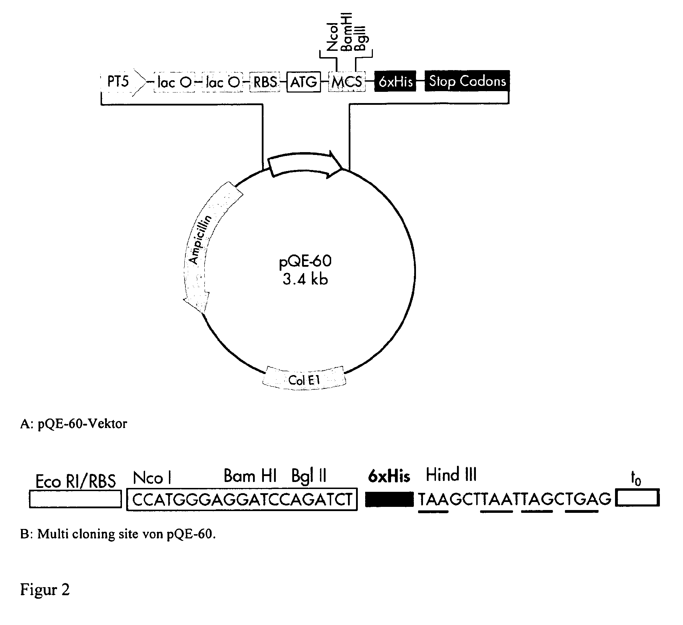

Mittels PCR wurde die DNA, die für das Protein YerE aus *Yersinia pseudotuberculosis* O*:VI* codiert, vervielfältigt. Dabei wurde außerdem am 5'-Ende eine Ncol-Schnittstelle, sowie am 3'-Ende eine Bglll-Schnittstelle eingefügt. Der Vektor pQE-60 (Figur 2, A) besitzt in seiner Multi Cloning Site (MCS) (Figur 2, B) diese beiden Schnittstellen. Sowohl der Vektor als auch das jeweilige PCR-Produkt wurden dann mit den beiden Restriktionsenzymen verdaut und im Anschluss wurde das jeweilige PCR-Produkt mit dem Vektor ligiert. Mit dem so erhaltenen Konstrukt wurden *E*. *coli* XL1blue zur Vektorproduktion und *E*.*coli* BL21 (DE3) Zellen zur Expression transformiert. Auf diese Weise wurde ein Fusionsprotein von YerE aus *Yersinia pseudotuberculosis* O*:VI* als C-terminal His-Tag tragendes Protein erhalten. Die Nucleotidsequenz von YerE ist im Sequenzprotokoll als SEQ ID NO:1 wiedergegeben.

### b) Expression

Die Expression erfolgte in *E.coli* BL21 (DE3) Zellen.
Die Zellen wurden in LB-Medium (100 µg/ml Ampicillin) bei 37 °C im Schüttelkolben inkubiert und nach IPTG-Zugabe (1.0 mM) über Nacht bei 24 °C exprimiert.

### c) Proteinreinigung

Die Proteinreinigung wurde über Immobilisierte Metallionen Affinitätschromatographie durchgeführt. Es wurden sowohl talon beads (Cobalt Ionen) als auch Nickel-NTA verwendet.

Aufgrund der sehr guten Expression des Proteins YerE (ca. 5% des Proteingehalts im Rohextrakt) sowie dem Ausbleiben von Nebenreaktionen wurde bei den Ansätzen mit Ketonen als Akzeptorsubstrate sowie bei dem Ansatz mit Oxobutyrat als Donorsubstrat mit dem lyophilisierten Rohextrakt gearbeitet.

### Beispiel 2: Pyruvat als Substrat für YerE

Ohne Anwesenheit eines Elektrophils (z.B. Aldehyd) bzw. eines weiteren Akzeptorsubstrates katalysiert YerE die Kondensation von Pyruvat zu Acetoin (Reaktionsgleichung IV). Diese Reaktivität wurde schon bei verschiedenen Pyruvatdecarboxylasen wie zum Beispiel aus *Zymomonas mobilis* ((*S*)-Acetoin) oder *Saccharomyces cerevisiae* ((*R*)-Acetoin) gefunden. 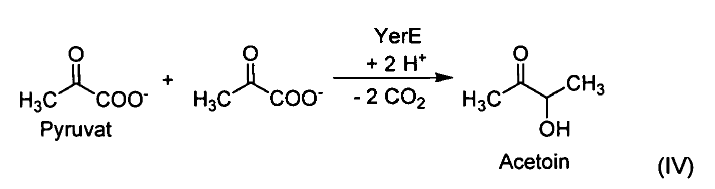 (IV) zeigt die YerE katalysierte Umsetzung von Pyruvat zu Acetoin. 1.5 ml Reaktionsvolumen, 50 mM Natriumpyruvat,
ee((S)-Acetoin) = 4.3%, chirale GC (Lipodex, 70 °C, Rₜ = 13.6 min (R), 18.4 (S)).

### Beispiel 3: Aldehyde als Akzeptoren

### Benzaldehyd und Derivate als Akzeptoren

In Gegenwart aromatischer Aldehyde werden ausgehend von Pyruvat mit YerE (R)-1-Phenyl-1-hydroxyketone mit guten Enantioselektivitäten (ee = 80 - 97%) gemäß Reaktionsgleichung (V) gebildet: 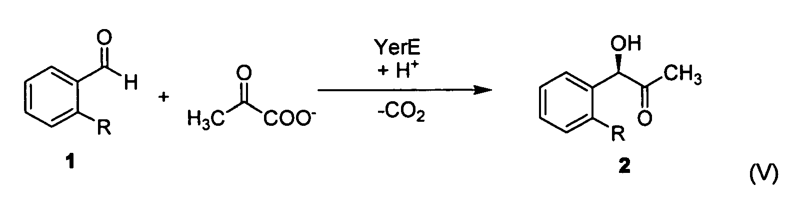 Bei dem Edukt (1) wurden die in der nachfolgenden Tabelle 1 aufgeführten Substituenten für R eingesetzt.

**Tabelle 1**

| Produkt (2) | GC-MS Umsatz [%] (nach 16 h) | HPLC (OB-Säule) *ee* [%] (nach 16 h) |
|---|---|---|
| **R = H** | 82 | 97 |
| **R = F** | 98 | 94 |
| **R = Cl** | 97 | 94 |
| **R = Br** | 97 | 94 |
| **R = I** | 95 | 80 |
| **R = CH₃** | 74 | 95 |

(V) zeigt die YerE katalysierte Umsetzungen mit Benzaldehyd und Derivaten. 1.5 ml Reaktionsvolumen, 20% DMSO, 20 mM Benzaldehyd-Derivat, 50 mM Natriumpyruvat, 300 µg YerE-Protein.

Dies zeigt, dass selbst sterisch gehinderte Verbindungen wie 2-Methylbenzaldehyd gute Akzeptoren sind.

### Beispiel 4: Cyclische Ketone als Akzeptoren

Für das gereinigte Enzym YerE konnte mit Cyclohexanon und methylierten Cyclohexanonen unter Decarboxylierung von Pyruvat eine intermolekulare Aldehyd-Keton Benzoin Reaktion nachgewiesen werden (Reaktionsgleichung VI).

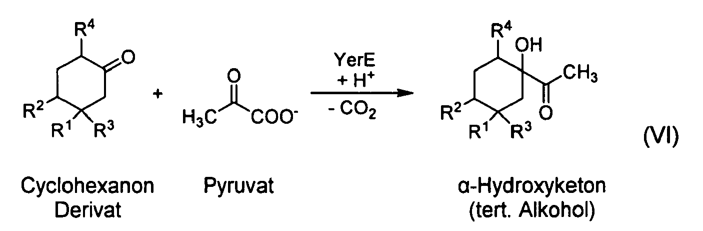

**Tabelle 2: YerE katalysierte Umsetzungen von Cyclohexanon und Derivaten.**

| **Edukt** | **R¹** | **R²** | **R³** | **R⁴** | **Umsatz [%] über GCMS** |
|---|---|---|---|---|---|
| Cyclohexanon | H | H | H | H | 60.7 |
| 2-Methylcyclohexanon | H | H | H | CH₃ | --- |
| 3-Methylcyclohexanon (rac.) | CH₃ | H | H | H | 48.6 |
| (*R*)-3-Methylcyclohexanon | CH₃ | H | H | H | 61.2 |
| 3,3-Dimethylcyclohexanon | CH₃ | H | CH₃ | H | --- |
| 4-Methylcyclohexanon | H | CH₃ | H | H | 66.9 + 8.8 (2 Isomere) |

Von einer Vielzahl an getesteten Verbindungen wurden nur 2-Methylcyclohexanon und 3,3-Dimethylcyclohexanon nicht von dem Enzym YerE umgesetzt.

Da bei der Umsetzung von 3-Methylcyclohexanon durch die enzymatische Reaktion ein Chiralitätszentrum aufgebaut wird, wurde diese Transformation herausgegriffen und näher untersucht. 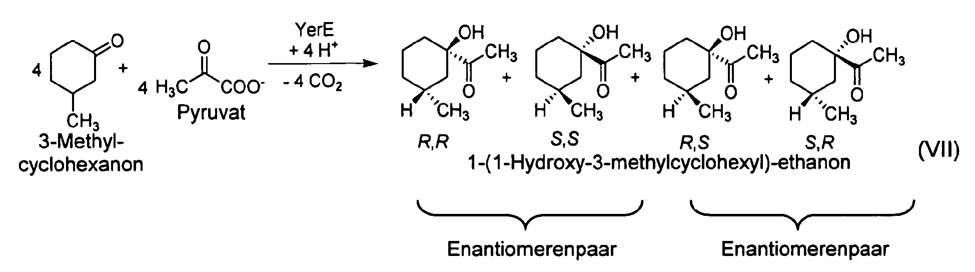 Reaktionsgleichung (VII): YerE katalysierte Umsetzung von 3-Methylcyclohexanon (rac.) mit Pyruvat.

Das enzymatische Produkt 1-(1-Hydroxy-3-methylcyclohexyl)-ethanon wurde zu Vergleichszwecken chemisch synthetisiert. Es zeigte sich, dass bei der chemischen Reaktion eines der beiden Enantiomerenpaare in großem Überschuss gegenüber dem zweiten gebildet wird. Aufgrund der Tatsache, dass sowohl für das *R,R*- als auch für das *S,S*-Diastereomer eine energetisch günstige Sesselkonformation existiert, bei der beide großen Substituenten (Acetyl- und Methylgruppe) eine äquatoriale Lage einnehmen, während für das *R,S*- und das *S,R*-Diastereomer keine derartige Konformation existiert, ist anzunehmen, dass es sich bei dem Enantiomerenpaar, welches bevorzugt gebildet wird, um das *R,R*- und das *S,S*-Diastereomer handelt.

Bei der enzymatischen Umsetzung wurde mittels NMR- und GCMS-Untersuchungen ausschließlich das über die chemische Synthese in großem Überschuss gebildete Enantiomerenpaar detektiert. Da der Versuch einer Trennung dieses Enantiomerenpaares mittels chiraler GC nicht zum gewünschten Erfolg führte, wurde (*S*)-3-Methylcyclohexanon, welches kommerziell nicht erhältlich ist, über einen Syntheseschritt chemisch synthetisiert, um zu überprüfen, ob sowohl (*R*)- als auch (*S*)-3-Methylcyclohexanon von dem Enzym YerE als Substrate akzeptiert werden. Hierbei zeigte sich, dass auch das (*S*)-Enantiomer durch das Protein in das entsprechende Diastereomer überführt wird (VIII). 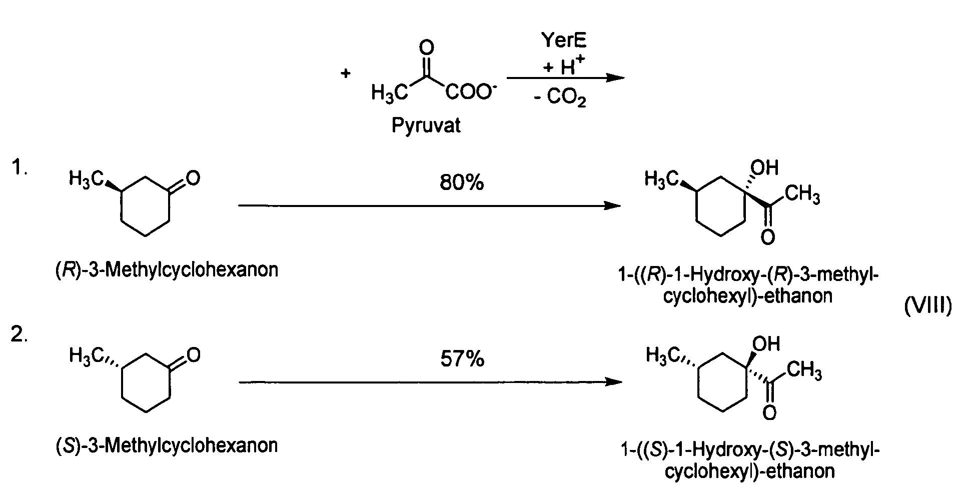 (VIII): Produktbildung in % über GC-MS bei cyclischen aliphatischen Ketonen als Akzeptorsubstrate. 1.5 ml Reaktionsvolumen, 20 mM Akzeptorsubstrat, 50 mM Natriumpyruvat, 1. 15.4 mg eingesetztes Gesamtprotein, 2. 15.4 mg eingesetztes Gesamtprotein.

Im Fall der Umsetzung von 3-Methylcyclohexanon sind demnach vermutlich kinetische und thermodynamische Effekte der Produktbildung und nicht die chirale Information des Enzyms YerE ausschlaggebend für die beobachtete Selektivität der Reaktion. Diese Beobachtung lässt darauf schließen, dass im Fall der physiologischen Reaktion des Proteins (s. Figur 1), der Aufbau des Stereozentrums an C4 ebenfalls durch die bereits am Ring gebundenen 3 Substituenten an C1, C2 und C5 und nicht durch das Enzym YerE vorgegeben wird.

### Beispiel 5: Cyclische aliphatische Ketone als Akzeptoren

Es zeigte sich, dass Pyruvat unter Decarboxylierung durch YerE-Katalyse an die cyclischen aliphatischen Ketone Cyclohexanon und 3-Methylcyclohexanon addiert wird (Reaktionsgleichung (IX)). 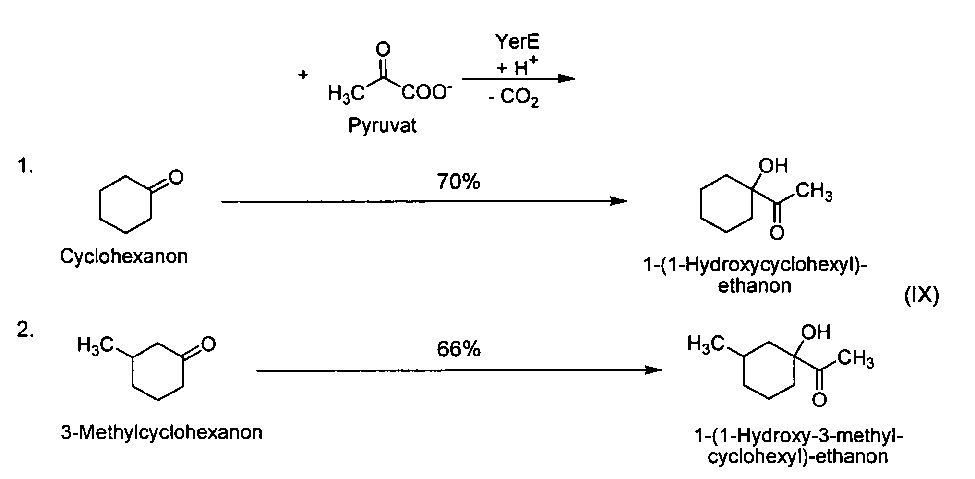 (IX): Produktbildung in % über GC-MS bei cyclischen aliphatischen Ketonen als Akzeptorsubstrate. 1.5 ml Reaktionsvolumen, 20 mM Akzeptorsubstrat, 50 mM Natriumpyruvat, 1. 15.1 mg eingesetztes Gesamtprotein, 2. 15.4 mg eingesetztes Gesamtprotein.

### Beispiel 6: Cyclisches Keton mit Heteroatom als Akzeptor

Um eine eventuelle enzymkatalysierte asymmetrische Reaktion untersuchen zu können, wurde das potentielle Substrat Tetrahydro-2*H*-pyran-3-on über eine zweistufige chemische Synthese synthetisiert, da in diesem Fall die Produktstabilität keinen Einfluss auf die Reaktion hat: Die Bildung beider möglicher Enantiomere ist hier, bezogen auf thermodynamische Aspekte, gleich wahrscheinlich.

Mittels GCMS-Untersuchung konnte in Gegenwart von Pyruvat eine nahezu vollständige Umsetzung des Substrates zu dem dargestellten Produkt 1-(3-Hydroxytetrahydro-2*H*-pyran-3-yl)-ethanon (X) detektiert werden. 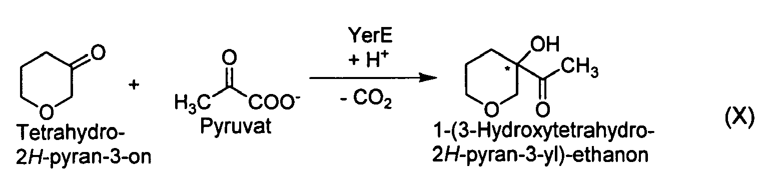 (X): YerE katalysierte Umsetzung von Tetrahydro-2*H*-pyran-3-on mit Pyruvat.

Für einen Ansatz im 50 ml Maßstab wurden 618 mg Gesamtprotein eingesetzt und 97% 1-(3-Hydroxytetrahydro-2*H*-pyran-3-yl)-ethanon im Roh NMR erhalten. Nach Aufreinigung über Säulenchromatographie(R_{f}(Dichlormethan / Aceton = 9:1) = 0.32): 49 mg 1-(3-Hydroxytetrahydro-2*H*-pyran-3-yl)-ethanon (34 % isolierte Ausbeute)

| | |
|---|---|
| GC-MS: | t*_{R}* = 6.53 min *m*/*z* = 144 ([M]⁺; 1.2%), 126 ([M - H₂O]⁺; 15.6%), 101 ([C₅H₉O₂]⁺; 100%), 83 ([C₅H₇O]⁺; 26.1%), 55 ([C₄H₇]⁺; 65.0%). |
| ¹H-NMR: | (400 MHz, CDCl₃, 25 °C): ö [ppm] = 1.54-1.68 (m, 1 H, CH₂), 1.71-1.79 (m, 1 H, CH₂), 1.89-2.00 (m, 2 H, CH₂), 2.32 (s, 3 H, CH₃), 3.41 (s, 1 H, OH), 3.47-3.72 (m, 3 H, CH₂), 3.89-3.98 (m, 1 H, CH₂). |
| ¹³C-NMR: | (100 MHz, CDCl₃, 25 °C): δ [ppm] = 21.1 (CH₂), 25.5 (CH₃), 31.3 (CH₂), 67.9 (CH₂), 72.6 (CH₂), 77.0 (Cq), 210.9 (C=O). |
| GC: | (Lipodex, 90 °C) Rₜ = 60.56 min (Hauptenantiomer), 59.28 min (*ent*). *ee* = 84% Der Enantiomerenüberschuss wurde mit dem chemisch synthetisierten Racemat als Referenz ermittelt. |
| Drehwert: | [α]_{D}²² = + 5.04 (α = + 0.0564, *c* = 1.1 g/100 ml, Chloroform). |

Mit dem chemisch synthetisierten Racemat als Referenz wurde ein Enantiomerenüberschuss von 84% des gereinigten 1-(3-Hydroxytetrahydro-2*H*-pyran-3-yl)-ethanon über chirale Gaschromathographie ermittelt.

### Beispiel 7: 1-Phenoxypropan-2-on als Akzeptor

Ausgehend von dem Substrat Tetrahydro-2*H*-pyran-3-on konnte durch Screening die offenkettige Verbindung 1-Phenoxypropan-2-on als weiteres Substrat des Enzyms YerE identifiziert werden. 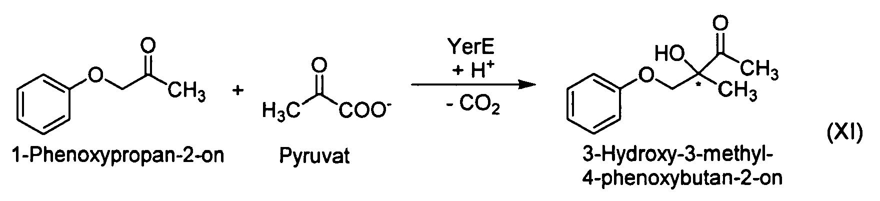 (XI): YerE katalysierte Umsetzung von 1-Phenoxypropan-2-on mit Pyruvat.

Im semipräparativen Maßstab wurden mit 14.9 mg Protein (= 745 µg YerE-Protein) 4.5 mg 1-Phenoxypropan-2-on zu 45% in 3-Hydroxy-3-methyl-4-phenoxybutan-2-on umgesetzt (GCMS).

Für einen Ansatz im 50 ml Maßstab wurden 588 mg Gesamtprotein eingesetzt und 48% 3-Hydroxy-3-methyl-4-phenoxybutan-2-on im Roh NMR erhalten. Nach Aufreinigung über Säulenchromatographie (R_{f}(Cyclohexan / Ethylacetat = 5:1) = 0.28): 56 mg 3-Hydroxy-3-methyl-4-phenoxybutan-2-on (29 % isolierte Ausbeute)

| | |
|---|---|
| GC-MS: | t*_{R}* = 9.26 min *m*/*z* = 194 ([M]⁺; 11.6%), 151 ([M - C₂H₃O]⁺; 100%), 133 ([C₉H₉C]⁺; 64.0%), 94 ([C₆H₆O]⁺; 57.3%), 77 ([C₆H₅]⁺; 36.0%). |
| ¹H-NMR: | (400 MHz, CDCl₃, 25 °C): ö [ppm] = 1.47 (s, 3 H, CH₃COH), 2.35 (s, 3 H, CH₃C=O), 4.00 (s, 1 H, OH), 4.02 (d, *J* = 9.4 Hz, 1 H, CH₂), 4.21 (d, *J* = 9.4 Hz, 1 H, CH₂), 6.85-6.93 (m, 2 H, ar-H), 6.96-7.03 (m, 1 H, ar-H), 7.25-7.34 (m, 2 H, ar-H). |
| ¹³C-NMR: | (100 MHz, CDCl₃, 25 °C): δ [ppm] = 21.7 (CH₃COH), 24.6 (CH₃C=O), 73.0 (CH₂), 78.3 (Cq), 114.6 (ar-C), 121.5 (ar-C), 129.5 (ar-C), 158.2 (ar-C), 210.3 (C=O). |
| HPLC: | (Chiracel AS-H, 25 °C, 0.75 ml·min⁻¹, *n*-Hexan/ 2-Propanol = 90:10) Rₜ = 10.15 min (*ent*) und 11.11 min (Hauptenantiomer). *ee* = 91% |
| Drehwert: | [α]_{D}²⁴ = - 43.7 (α = -0.5244, *c* = 1.2 g/100 ml, Chloroform). |

Der Enantiomerenüberschuss von 91% des gereinigten 3-Hydroxy-3-methyl-4-phenoxybutan-2-ons wurde über chirale HPLC mit dem chemisch synthetisierten Racemat als Referenz ermittelt.

### Beispiel 8: Offenkettige aliphatische Ketone als Akzeptoren

Pyruvat wird unter YerE-Katalyse ebenfalls an offenkettige aliphatische Ketone addiert (Gleichung XII). 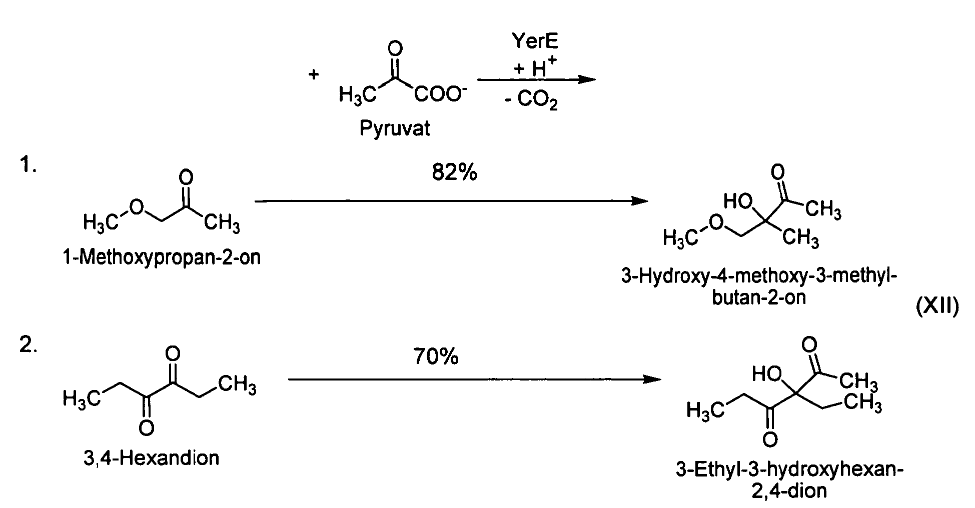 (XII): Produktbildung in % über GC-MS bei offenkettigen aliphatischen Ketonen als Akzeptorsubstrate. 1.5 ml Reaktionsvolumen, 20 mM Akzeptorsubstrat, 50 mM Natriumpyruvat, 1. 15.1 mg eingesetztes Gesamtprotein, 2. 18.2 mg eingesetztes Gesamtprotein.

Für einen Ansatz im 50 ml Maßstab wurden 690 mg Gesamtprotein eingesetzt.
Aufgrund des Zusammenfallens der Edukt- und Produkt-Signale kann hier keine Angabe des prozentualen Anteils von 3-Ethyl-3-hydroxyhexan-2,4-dion im Roh NMR erfolgen. Nach Aufreinigung über Säulenchromatographie (R_{f}(Cyclohexan / Ethylacetat = 5:1) = 0.50): 53 mg 3-Ethyl-3-hydroxyhexan-2,4-dion (34 % isolierte Ausbeute)

| | |
|---|---|
| GC-MS: | t*_{R}* = 5.71 min *m*/*z* = 158 ([M]⁺; 0.6%), 116 ([C₆H₁₂O₂]⁺; 59.1%), 102 ([C₅H₁₀O₂]⁺; 99.8%), 87 ([C₄H₇O₂]⁺; 73.2%), 57 ([C₃H₅O]⁺; 100%). t*_{R}* = 5.79 min *m*/*z* = 158 (0.4%), 116 (9.5%), 102 (100%), 87 (52.2%), 57 (94.3%). |
| ¹H-NMR: | (400 MHz, CDCl₃, 25 °C): ö [ppm] = 0.83 (t, *J* = 7.4 Hz, 3 H, CH₃), 1.04 (t, *J* = 7.3 Hz, 3 H, CH₃), 2.03 (q, *J* = 7.4 Hz, 2 H, CH₂), 2.25 (s, 3 H, CH₃), 2.44-2.60 (m, 1 H, CH₂), 2.68-2.82 (m, 1 H, CH₂), 4.66 (s, 1 H, OH). |
| ¹³C-NMR: | (100 MHz, CDCl₃, 25 °C): δ [ppm] = 5.2 (CH₃), 5.2 (CH₃), 25.1 (CH₃), 29.7 (CH₂), 30.9 (CH₂), 91.0 (Cq), 207.8 (C=O), 210.2 (C=O). |
| GC: | (Lipodex D, 90 °C) Rₜ = 10.68 min (Hauptenantiomer), 12.02 min (*ent*). *ee* = 84% |
| Drehwert: | [α]_{D}²² = - 15.3 (α = - 0.1778, *c* = 1.2 g/ 100 ml, Chloroform). |

### Beispiel 9: Ketone mit aromatischen Substituenten als Akzeptoren

Pyruvat wird unter YerE-Katalyse ebenfalls an Ketone mit aromatischen Substituenten addiert (Reaktionsgleichung XIII.1 - XIII.5). 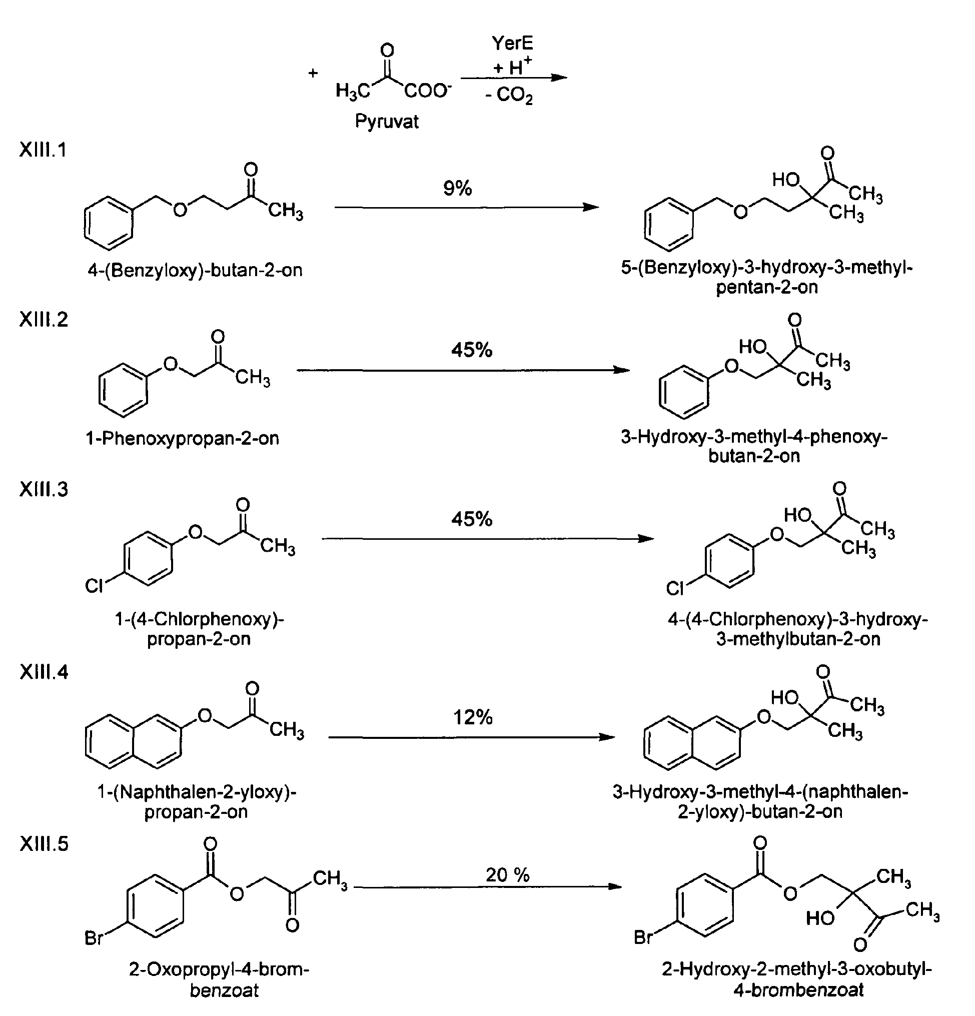 Gleichungen XIII.1 - XIII.5: Produktbildung in % über GC-MS bei aromatischen Ketonen als Akzeptorsubstrate. 1.5 ml Reaktionsvolumen, 20 mM Akzeptorsubstrat, 50 mM Natriumpyruvat, bei XIII.3., XIII.4. und XIII.5. 20 % DMSO.
XIII.1. 18.3 mg eingesetztes Gesamtprotein, XIII.2. 14.9 mg eingesetztes Gesamtprotein, XIII.3. 17.5 mg eingesetztes Gesamtprotein, XIII.4. 15.0 mg eingesetztes Gesamtprotein, XIII.5. 18.3 mg eingesetztes Gesamtprotein.

### Beispiel 10: Variation des Donorsubstrates

Als weiteres Donorsubstrat zeigt 2-Oxobutyrat Aktivität in Gegenwart von Carbonylen. 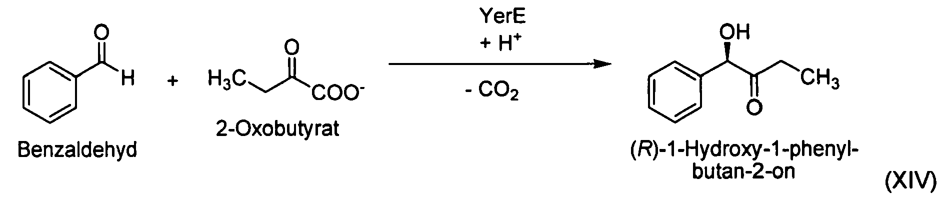 Gleichung XIV: 2-Oxobutyrat als Donorsubstrat. Produktbildung über GC-MS: > 99%.
1.5 ml Reaktionsvolumen, 20 mM Akzeptorsubstrat, 50 mM 2-Oxobutyrat, 19.7 mg eingesetztes Gesamtprotein
ee((*R*)-1-Hydroxy-1-phenylbutan-2-on) > 98%, chirale HPLC (Chiralcel OD-H, 40 °C, 0.5 ml·min⁻¹, n-Hexan/ 2-Propanol = 95:5, Rₜ = 20.4 min (*R*)).

### Beispiel 11: Reaktionen im 50 ml Massstab

### a) Allgemeine Ansatzbedingungen

Es wurden jeweils 20 mM Akzeptorsubstrat und 50 mM Natriumpyruvat eingesetzt. Das Proteinlyophilisat wurde in 50 mM KPi-Puffer gelöst. Zu dieser Lösung wurde das Akzeptorsubstrat in Reinform und das Natriumpyruvat, gelöst in 50 mM KPi-Puffer zugegeben. Bei dem enzymatischen Ansatz mit 1-Phenoxypropan-2-on wurden zusätzlich 2.5 ml Methyl-*tert*-butylether (MTBE) (5%) als Lösungvermittler eingesetzt. Im Anschluss wurde die Reaktionslösung mit 50 mM KPi-Puffer auf 50.0 ml aufgefüllt.

Die Proteinlyophilisate enthielten 35 - 40% eingesetztes Gesamtprotein.
Zusammensetzung des KPi-Puffers:
50 mM KPi
1.5 mM MgCl₂
0.05 mM ThDP
0.02 mM FAD

### b) Aufarbeitung

Die Reaktionslösung wurde bei den Ansätzen mit Tetrahydro-2*H*-pyran-3-on und 3,4-Hexandion mit Methyl-*tert*-butylether (MTBE), bei dem Ansatz mit 1-Phenoxy-propan-2-on mit Ethylacetat, extrahiert. Bei dem Ansatz mit 3,4-Hexandion erfolgte zuvor noch eine Zentrifugation der Reaktionslösung zur Abtrennung des ausgefallenen Proteins. Die organische Phase wurde über wasserfreiem Natriumsulfat getrocknet und das Lösungsmittel unter vermindertem Druck entfernt.

Das Rohprodukt wurde über Säulenchromatographie an Kieselgel 60 gereinigt. Für die Reinigung der Rohprodukte, die ausgehend von Tetrahydro-2*H*-pyran-3-on und 1-Phenoxypropan-2-on erhalten wurden, erfolgte die Reinigung an Salzsäure-behandeltem Kieselgel 60.

### Beispiel 12: Enantiomerenüberschuss

Der Enantiomerenüberschuss der durch YerE Katalyse entstandenen Produkte wurde über chirale GC-FID und chirale HPLC-DAD bestimmt.

### Enantiomerenüberschuss der durch YerE Katalyse entstandenen Produkte:

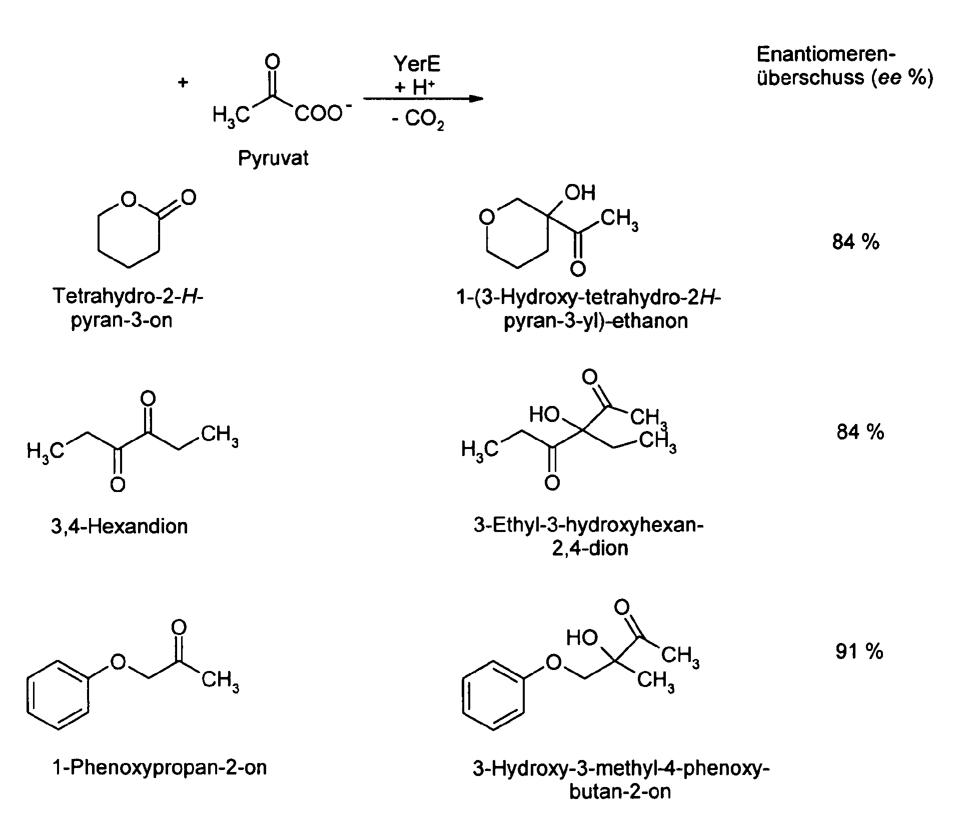

### Beispiel 13 (Vergleichsbeispiel):

### a) Chemische Synthese des razemischen 1-(3-Hydroxytetrahydro-2H-pyran-3-yl)-ethanon:

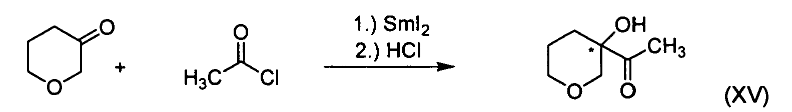

(XV): Chemische Synthese des razemischen 1-(3-Hydroxytetrahydro-2*H*-pyran-3-yl)-ethanon als Referenz.

| | |
|---|---|
| GC-MS: | t*_{R}* = 6.53 min *m*/*z* = 144 ([M]⁺; 1.2%), 126 ([M - H₂O]⁺; 15.6%), 101 ([C₅H₉O₂]⁺; 100%), 83 ([C₅H₇O]⁺; 26.1%), 55 ([C₄H₇]⁺; 65.0%). |
| ¹H-NMR: | (400 MHz, CDCl₃, 25 °C): δ [ppm] = 1.54-1.68 (m, 1 H, CH₂), 1.71-1.79 (m, 1 H, CH₂), 1.89-2.00 (m, 2 H, CH₂), 2.32 (s, 3 H, CH₃), 3.41 (s, 1 H, OH), 3.47-3.72 (m, 3 H, CH₂), 3.89-3.98 (m, 1 H, CH₂). |
| ¹³C-NMR: | (100 MHz, CDCl₃, 25 °C): δ [ppm] = 21.1 (CH₂), 25.5 (CH₃), 31.3 (CH₂), 67.9 (CH₂), 72.6 (CH₂), 77.0 (Cq), 210.9 (C=O). |
| GC: | (Lipodex, 90 °C) Rₜ = 60.56 min, 59.28 min. Razemat |

### b) Chemische Synthese des razemischen 3-Hydroxy-3-methyl-4-phenoxybutan-2-on:

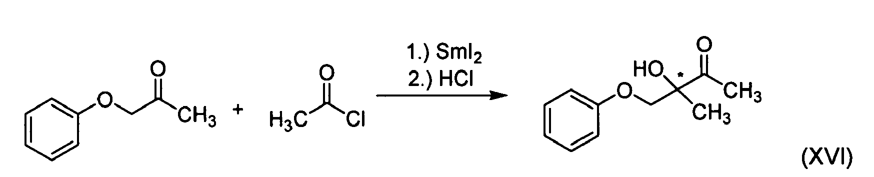

(XVI): Chemische Synthese des razemischen 3-Hydroxy-3-methyl-4-phenoxybutan-2-on als Referenz.

| | |
|---|---|
| GC-MS: | t*_{R}* = 9.26 min *m*/*z* = 194 ([M]⁺; 11.6%), 151 ([M - C₂H₃O]⁺; 100%), 133 ([C₉H₉O]⁺; 64.0%), 94 ([C₆H₆O]⁺; 57.3%), 77 ([C₆H₅]⁺; 36.0%). |
| ¹H-NMR: | (400 MHz, CDCl₃, 25 °C): δ [ppm] = 1.47 (s, 3 H, CH₃COH), 2.35 (s, 3 H, CH₃C=O), 4.00 (s, 1 H, OH), 4.02 (d, *J* = 9.4 Hz, 1 H, CH₂), 4.21 (d, *J* = 9.4 Hz, 1 H, CH₂), 6.85-6.93 (m, 2 H, ar-H), 6.96-7.03 (m, 1 H, ar-H), 7.25-7.34 (m, 2 H, ar-H). |
| ¹³C-NMR: | (100 MHz, CDCl₃, 25 °C): ö [ppm] = 21.7 (CH₃COH), 24.6 (CH₃C=O), 73.0 (CH₂), 78.3 (Cq), 114.6 (ar-C), 121.5 (ar-C), 129.5 (ar-C), 158.2 (ar-C), 210.3 (C=O). |
| HPLC: | (Chiracel AS-H, 25 °C, 0.75 ml·min⁻¹, *n*-Hexan/ 2-Propanol = 90:10) Rₜ = 10.15 min und 11.11 min. Razemat |

### Beispiel 14: Absolute Konfiguration

### a) Aldehyde als Akzeptoren

Zur Bestimmung der absoluten Konfiguration der ausgehend von Benzaldehydderivaten gebildeten enzymatischen Produkte wurde die Umsetzung von 2-Chlorbenzaldehyd (Akzeptor) mit Pyruvat (Donor) herausgegriffen und das Produkt im präparativen Massstab isoliert.

### Synthese von 1-(2-Chlorphenyl)-1-hydroxypropan-2-on

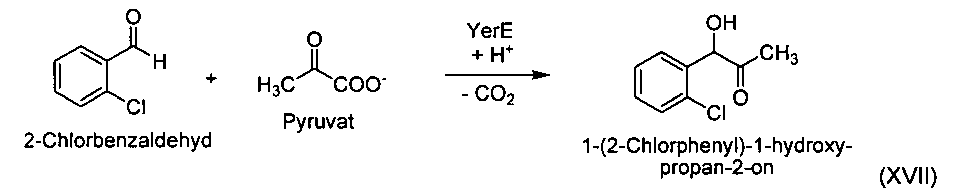

(XVII): YerE katalysierte Umsetzung von 2-Chlorbenzaldehyd zu 1-(2-Chlorphenyl)-1-hydroxypropan-2-on.
10 mg YerE-Protein
90% 1-(2-Chlorphenyl)-1-hydroxypropan-2-on im Roh NMR
Nach Aufreinigung über Säulenchromatographie
(R_{f}(Cyclohexan / Ethylacetat = 5:1) = 0.25):
111 mg 1-(2-Chlorphenyl)-1-hydroxypropan-2-on (60% isolierte Ausbeute)

| | |
|---|---|
| GC-MS: | t*_{R}* = 8.80 min *m*/*z* = 184 ([M]⁺; 0.13%), 143 ([C₆H₄ClCOH]⁺; 29%), 141 ([C₆H₄ClCOH]⁺; 100%), 77 ([C₆H₅]⁺; 76%), 51 ([C₄H₃]⁺; 12%). |
| ¹H-NMR: | (400 MHz, CDCl₃, 25 °C): δ [ppm] = 2.16 (s, 3 H, CH₃), 4.37 (d, *J* = 4.3 Hz, 1 H, OH), 5.61 (d, *J* = 4.3 Hz, 1 H, CH), 7.29 - 7.33 (m, 3 H, ar-H), 7.42 - 7.47 (m, 1 H, ar-H). |
| ¹³C-NMR: | (100 MHz, CDCl₃, 25 °C): δ [ppm] = 25.3 (CH₃), 76.5 (CHOH), 127.5 (C-5'), 128.9, 129.9, 130.0 (C-3',4',6'), 133.4 (C*_{q}*), 135.6 (C*_{q}*), 206.3 (C=O). |
| HPLC: | (Chiracel OB, n-Hexan / 2-Propanol = 90 : 10, 0.75 ml · min⁻¹, 20 °C) Rₜ = 14.62 min (Hauptenantiomer), Rₜ = 12.58 min (*ent*). *ee* = 73.9 % |
| Drehwert: | [α]_{D}²³= - 254.4 (α = - 3.0913, *c* = 1.2 g/ 100 ml, Chloroform). |
| Mol-CD: | Zur Bestimmung der absoluten Konfiguration des Überschussenantiomeren wurde das CD-Spektrum von 1-(2-Chlorphenyl)-1-hydroxy-propan-2-on mit dem von (*R*)-Phenylacetylcarbinol ((*R*)-PAC) verglichen. Das (*R*)-PAC wurde hierfür über einen enzymatischen Ansatz unter Nutzung des Enzyms Pyruvatdecarboxylase aus *Saccharomyces cerevisiae* (*Sc*PDC) gewonnen. |

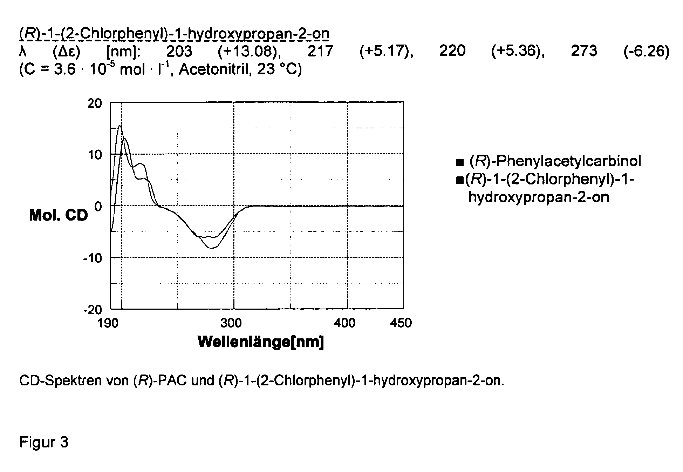

Das CD-Spektrum ist in Figur 3 gezeigt.

Da es sich bei (*R*)-PAC und der mit YerE synthetisierten Verbindung 1-(2-Chlorphenyl)-1-hydroxypropan-2-on um sehr ähnlich aufgebaute Moleküle mit vergleichbaren UV-Chromophoren (Aromat: π→ π*, Carbonylfunktion: n→ π*) handelt, welche die gleiche Konformation einnehmen, kann aus der großen Ähnlichkeit zwischen den CD-Spektren beider Verbindungen (gleiche Minima und Maxima) geschlossen werden, dass es sich bei dem überschüssigen Enantiomer der synthetisierten Verbindung um das (*R*)-1-(2-Chlorphenyl)-1-hydroxypropan-2-on handelt.

### b) Ketone als Akzeptoren

Über die absolute Konfiguration der dargestellten enzymatischen Produkte die ausgehend von Ketonen als Akzeptoren erhalten wurden, kann zum jetzigen Zeitpunkt noch keine Aussage getroffen werden, da in diesen Fällen keine Literaturdaten zu entsprechenden Referenzsubstanzen existieren, die eine Zuordnung der absoluten Konfiguration erlauben würden.

### Optische Rotation

Drehwert der Produkte durch YerE Katalyse:

| | |
|---|---|
| 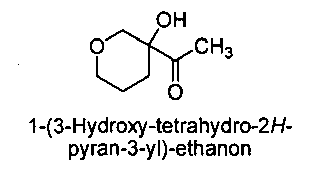 | Drehwert: [α]_{D}²² = + 5.04 (α = + 0.0564, *c* = 1.1 g/ 100 ml, Chloroform). |
| 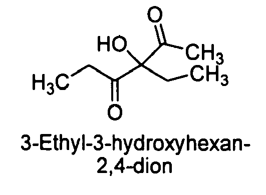 | Drehwert: [α]_{D}²² = - 15.3 (α = - 0.1778, *c* = 1.2 g/ 100 ml, Chloroform). |
| 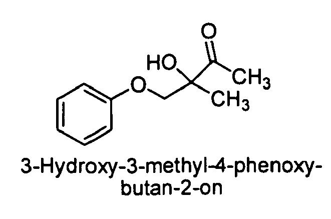 | Drehwert: [α]_{D}²⁰ = - 43.7 (α = - 0.5244, *c* = 1.2 g/ 100 ml, Chloroform). |

## Patentansprüche

1. Verfahren zur Ausbildung einer kovalenten Bindung zwischen zwei Gruppen mit Carbonylresten gemäß der vorliegenden Reaktionsgleichung: 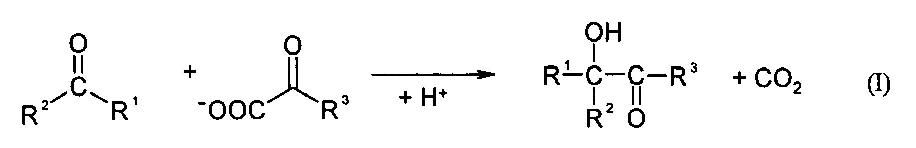 worin
R¹ Wasserstoff, eine Carboxylgruppe oder C₁-C₆ Alkyl ist und
R² ein gegebenenfalls mit Halogen und / oder C₁-C₆ - Alkyl mono-oder disubstituierter aromatischer oder heteroaromatischer Rest oder ein Substituent mit der Formel -AB-C-D ist, wobei A und C jeweils C₁-C₆ Alkyl, Carbonyl oder eine kovalente Bindung ist, B ein Heteroatom, ausgewählt aus Sauerstoff und Schwefel und D ein aliphatischer Rest mit 1 bis 6 Kohlenstoffatomen, ein gegebenenfalls substituierter aromatischer oder heteroaromatischer Rest ist,
oder R¹ und R² zusammen einen cyclischen, gegebenenfalls mit einem C₁-C₆ Alkylrest und/oder mit einem Hydroxyrest mono- oder disubstituierten Alkylrest bildet, der gegebenenfalls ein Heteroatom, nämlich Sauerstoff oder Schwefel enthalten kann, und
R³ Wasserstoff oder C₁-C₆ Alkyl ist,
**dadurch gekennzeichnet,dass** die Reaktion durch das Enzym YerE katalysiert wird.

2. Verfahren nach Anspruch 1, **dadurch gekennzeichnet, dass** die Gruppe mit der Formel 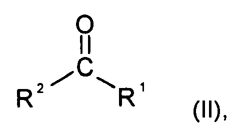 bei der Reaktionsgleichung (I) ein Akzeptor ist, bei dem R¹ die Bedeutung Wasserstoff, eine Carboxylgruppe oder C₁-C₆-Alkyl hat und R² ein gegebenenfalls mit einem Heteroatom und/oder C₁-C₆ Alkyl mono- oder disubstituierter aromatischer Rest ist.

3. Verfahren nach Anspruch 1, **dadurch gekennzeichnet, dass** die Gruppe mit der Formel 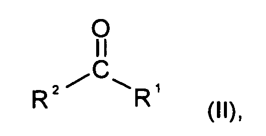 bei der Reaktionsgleichung (I) ein Akzeptor ist, bei dem R¹ die Bedeutung C₁-C₆ Alkyl hat und R² ein Substituent mit der Formel -A-B-C-D ist, wobei A und C die Bedeutung -(CH₂)-ₙ mit n = 1 bis 6 hat oder eine kovalente Bindung ist, B ein Heteroatom, ausgewählt unter Sauerstoff und Schwefel, oder eine kovalente Bindung ist, und D ein mono- oder disubstituierter aromatischer oder heteromaromatischer Rest ist.

4. Verfahren nach Anspruch 3, **dadurch gekennzeichnet, dass** R² ausgewählt ist aus Gruppen der Formeln: 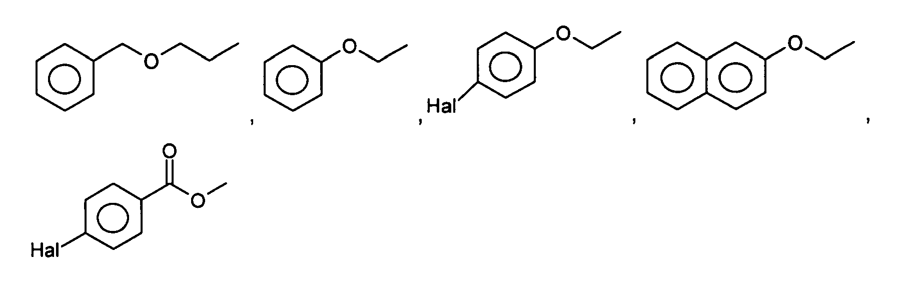 wobei Hal ausgewählt ist aus F, Cl, Br, oder I.

5. Verfahren nach einem der vorhergehenden Ansprüche, **dadurch gekennzeichnet, dass** die Gruppe mit der Formel 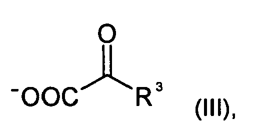 ein Donorsubstrat ist, bei dem R³ die Bedeutung Wasserstoff oder C₁-C₆ Alkyl hat.

6. Verfahren nach einem der vorhergehenden Ansprüche, **dadurch gekennzeichnet, dass** die Gruppe mit der Formel (II) 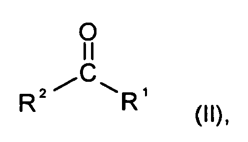 und die Gruppe mit der Formel (III) 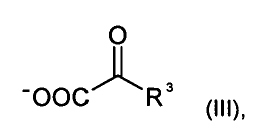 vor der Reaktion gemäß Reaktionsgleichung (I) nicht miteinander kovalent verbunden sind.

7. Verfahren nach einem der Ansprüche 1-5, **dadurch gekennzeichnet, dass** die Gruppe mit der Formel (II) 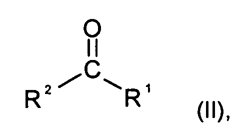 und die Gruppe mit der Formel (III) 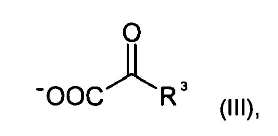 schon vor Durchführung der Reaktion gemäß Reaktionsgleichung (I) miteinander kovalent verbunden sind, wobei Rest R¹ oder Rest R² kovalent mit Rest R³ verbunden sind und die kovalente Bindung gemäß Reaktionsgleichung (I) intermolekular erfolgt.

8. Verfahren nach einem der vorhergehenden Ansprüche, **dadurch gekennzeichnet, dass** das Enzym YerE in Form eines Rohextraktes oder partiell gereinigten Rohextrakts eingesetzt wird, der wenigstens 5 Gew.-% Enzym YerE, bezogen auf das Gesamtprotein, enthält.

9. Verfahren nach einem der Ansprüche 1-7, **dadurch gekennzeichnet, dass** das Enzym YerE in Form einer gereinigten Enzympräparation eingesetzt wird, **dadurch gekennzeichnet, dass** die Enzympräparation wenigstens 80 Gew.-% YerE-Enzym, bezogen auf das eingesetzte Gesamtprotein, enthält.

10. Verfahren nach einem der vorhergehenden Ansprüche, **dadurch gekennzeichnet, dass** das Enzym YerE rekombinant in einem Wirtsorganismus hergestellt wird, der nicht zum Genus Yersinia gehört.

11. Verfahren nach einem der vorhergehenden Ansprüche, **dadurch gekennzeichnet, dass** das Enzym YerE eine Aminosäuresequenz aufweist, die zu wenigstens 70 % homolog ist zu der SEQ ID NO:2.

12. Verwendung eines isolierten Enzyms YerE zur Ausbildung einer kovalenten Kohlenstoff-Kohlenstoff-Bindung zwischen zwei Gruppen, die jeweils eine Carbonyl-Gruppe an den zu verbindenden Kohlenstoff-Atomen aufweisen, wobei das dabei entstehende Produkt mit einem hohen Enantiomeren-Überschuß entsteht.
